# EUROPEAN PATENT SPECIFICATION

(11) **EP 4 192 534 B1**
(45) Date of publication and mention of the grant of the patent: **06.11.2024**
(21) Application number: 21755070.6
(22) Date of filing: 03.08.2021
(51) Int. Cl.: A61L 15/32

(54) **METHOD OF MAKING COLLAGEN WOUND DRESSINGS**
VERFAHREN ZUR HERSTELLUNG VON KOLLAGEN-WUNDVERBÄNDEN
MÉTHODE DE FABRICATION DE PANSEMENTS EN COLLAGÈNE

(30) Priority: 04.08.2020 US 202063060939 P
(43) Date of publication of application: 14.06.2023
(73) Proprietor: Solventum Intellectual Properties Company, Maplewood, MN 55144 (US)
(72) Inventor: BAKER, Bryan A., Saint Paul, Minnesota 55133-3427 (US); NELSON, Caleb T., Saint Paul, Minnesota 55133-3427 (US); SWANSON, Steven P., Saint Paul, Minnesota 55133-3427 (US)
(74) Representative: Mathys & Squire
(86) International application number: PCT/IB2021/057115
(87) International publication number: WO 2022/029631

(56) References cited:
- EP-A2- 0 901 795
- US-A1- 2018 064 580
- ESWARAMOORTHY NITHYA ET AL: "Plasma treatments of dressings for wound healing: a review", BIOPHYSICAL REVIEWS, SPRINGER, DE, vol. 9, no. 6, 2 October 2017 (2017-10-02), pages 895 - 917, XP036885882, ISSN: 1867-2450, [retrieved on 20171002], DOI: 10.1007/S12551-017-0327-X

## Description

### BACKGROUND

Collagen dressings are used as wound care products. These products are primarily derived from bovine collagen sources, particularly bovine skin, and processed via acid or enzymatic extraction methods into purified and largely type I collagen material.

EP 0 901 795 A1 describes solid bioabsorbable materials comprising a weak, water soluble acid buffer system dissolved or dispersed in a protein matrix in an amount of at least 0.1 mmol/g based on the weight of the material, wherein the buffer system comprises a weak, water-soluble, physiologically acceptable acid and a water-soluble, physiologically acceptable conjugate base of a weak acid and wherein the material is preferably used as or in wound dressings in order to maintain wound surfaces at acid pH, preferably pH 3.5 to 6.5, and preferably a collagen sponge having a solid hydrogen phosphate/dihydrogen phosphate buffer dispersed therein.

US 2018/064580 A1 describes a method of preparing a crosslinked, collagen-based wound care dressing, comprising: (a) immersing a sample of fibrous and/or non-fibrous collagen in a buffered acidic, aqueous solution comprising an alcohol, wherein the immersion of the sample of fibrous and/or non-fibrous collagen is conducted prior to crosslinking of the collagen; (b) subsequent to step a), contacting the collagen in a separate solution with a catalytic component comprising 1-ethyl-3-[3-dimethylaminopropyl] carbodiimide hydrochloride (EDCI) for a time at least sufficient to effect reaction between amino and carboxyl groups present on the collagen and to yield crosslinked collagen, wherein the crosslinked collagen is resistant to pronase degradation when subjected to a pronase degradation test for at least five hours; and (c) drying the crosslinked collagen to yield a porous, crosslinked collagen article wherein the porous, crosslinked collagen article demonstrates a pore size of 10-500 microns.

The article "Plasma treatments of dressings for wound healing: a review" published in Biophysical Reviews on December 2017 by N. Eswaramoorthy (XP036885882) is a review which covers the use of plasma technology relevant to the preparation of dressings for wound healing.

Collagen dressings can improve collagen and fibroblast activity at a wound site. There is a need to provide better collagen dressings in a form useful for treating wounds.

### SUMMARY

According to the invention , the present disclosure provides a method of making an article, comprising, dissolving collagen in an acid solution; forming a collagen matrix; and reducing pH value of the collagen matrix by a first plasma treatment; wherein the article is a wound dressing.

Various aspects and advantages of exemplary embodiments of the present disclosure have been summarized. The above Summary is not intended to describe each illustrated embodiment or every implementation of the present disclosure. Further features and advantages are disclosed in the embodiments that follow. The Drawings and the Detailed Description that follow more particularly exemplify certain embodiments using the principles disclosed herein.

### DEFINITIONS

For the following defined terms, these definitions shall be applied for the entire Specification, including the claims, unless a different definition is provided in the claims or elsewhere in the Specification based upon a specific reference to a modification of a term used in the following definitions:
The terms "about" or "approximately" with reference to a numerical value or a shape means +/- five percent of the numerical value or property or characteristic, but also expressly includes any narrow range within the +/- five percent of the numerical value or property or characteristic as well as the exact numerical value. For example, a temperature of "about" 100°C refers to a temperature from 95°C to 105°C, but also expressly includes any narrower range of temperature or even a single temperature within that range, including, for example, a temperature of exactly 100°C. For example, a viscosity of "about" 1 Pa-sec refers to a viscosity from 0.95 to 1.05 Pa-sec, but also expressly includes a viscosity of exactly 1 Pa-sec. Similarly, a perimeter that is "substantially square" is intended to describe a geometric shape having four lateral edges in which each lateral edge has a length which is from 95% to 105% of the length of any other lateral edge, but which also includes a geometric shape in which each lateral edge has exactly the same length.

The term "substantially" with reference to a property or characteristic means that the property or characteristic is exhibited to a greater extent than the opposite of that property or characteristic is exhibited. For example, a substrate that is "substantially" transparent refers to a substrate that transmits more radiation (e.g. visible light) than it fails to transmit (e.g. absorbs and reflects). Thus, a substrate that transmits more than 50% of the visible light incident upon its surface is substantially transparent, but a substrate that transmits 50% or less of the visible light incident upon its surface is not substantially transparent.

The terms "a", "an", and "the" include plural referents unless the content clearly dictates otherwise. Thus, for example, reference to a material containing "a compound" includes a mixture of two or more compounds.

### BRIEF DESCRIPTION OF THE DRAWINGS

The disclosure may be more completely understood in consideration of the following detailed description of various embodiments of the disclosure in connection with the accompanying figures, in which:
FIG. 1 is a schematic cross-section of an illustrative embodiment of an article suitable for a wound dressing in the form of a sheet;
FIG. 2 is a schematic cross-section of an illustrative embodiment of an article suitable for a wound dressing in the form of a foam;
FIG. 3 is a schematic cross-section of an illustrative embodiment of an article suitable for a wound dressing;
FIG. 4 is a schematic cross-section of an illustrative embodiment of an article suitable for a wound dressing comprising a carrier layer;

While the above-identified drawings, which may not be drawn to scale, set forth various embodiments of the present disclosure, other embodiments are also contemplated, as noted in the Detailed Description. In all cases, this disclosure describes the presently disclosed invention by way of representation of exemplary embodiments and not by express limitations.

### DETAILED DESCRIPTION

Before any embodiments of the present disclosure are explained in detail, it is understood that the invention is not limited in its application to the details of use, construction, and the arrangement of components set forth in the following description. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter and equivalents thereof as well as additional items. It is understood that other embodiments may be utilized and structural or logical changes may be made without departing from the scope of the present disclosure.

As used in this Specification, the recitation of numerical ranges by endpoints includes all numbers subsumed within that range (e.g. 1 to 5 includes 1, 1.5, 2, 2.75, 3, 3.8, 4, and 5, and the like).

Unless otherwise indicated, all numbers expressing quantities or ingredients, measurement of properties and so forth used in the Specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The articles and methods of the description are useful as wound dressings and in the healing of wounds (both acute and chronic). Without wishing to be bound by theory, the articles and methods of the description are believed to improve wound healing in part by lowering the pH of the wound environment. Lowering the pH of the wound environment (such as the exposed wound surface and wound fluid) can improve wound healing by reducing detrimental protease activity in a wound; inhibiting the growth of pathogenic bacteria in an infected wound; and increasing oxygen levels in wound tissue. Outcomes of improved wound healing can include faster wound healing and\or more complete wound healing.

In some embodiments, an article is described. The article can be a wound dressing. The article can include a more than 50 wt% collagen matrix. In some embodiments, the collagen matrix has a pH value from 2 to 6, from 3 to 6, from 2 to 5, from 3 to 5, from 2 to 4.5, from 2 to 4, or from 2 to 3. The collagen matrix can be plasma treated.

In some embodiments, the collagen matrix is a conformable matrix. In some embodiments, the article is a conformable wound dressing. A conformable wound dressing means that the dressing can be shaped to the contours of a wound bed, enabling interaction of the dressing with a non-uniform surface found in different kinds of wounds.

According to the invention, a method of forming an article is described. The method comprises dissolving collagen in an acid solution; forming a collagen matrix; and reducing pH value of the collagen matrix by a first plasma treatment. In some embodiments, the article is a wound dressing. In some embodiments, the first plasma treatment is substantially at atmospheric pressure. In some embodiments, the first plasma treatment can include introducing an input gas comprising oxygen, nitrogen, and water vapor. In some embodiments, the method can include a second plasma treatment after the first plasma treatment. In some embodiments, the second plasma treatment can include introducing an oxygen plasma at sub atmospheric pressure.

Any suitable sources of collagen can be used. For example, the species from which the collagen is obtained could be human, bovine, porcine, or other animal sources. Collagen can also be obtained from recombinant sources. Collagen can also be obtained commercially as aqueous solutions, and the concentrations of these solutions may vary. Alternatively, collagen can be provided in lyophilized form and stored at very low temperatures. In some embodiments, collagen can be dissolved in acetic acid. In some embodiments, the amount of collagen is at least 1 mg/mL and typically no greater than 120 mg/mL.

In typical embodiments, a plasma treatment is used to reduce pH value to the desired pH range. In some embodiments, the collagen matrix has a pH value from 2 to 5, from 2 to 4, or from 2 to 3. In some embodiments, the collagen matrix has a pH value less than 6, 5, 4.5, 4, 3.5, 3, or 2.5. In some embodiments, the collagen matrix has a pH value greater than 1.2, 1.5, 1.8, 1.9, 2, 2.5, or 3. The pH of the collagen matrix can be determined according to either 'pH Test Method A' or 'pH Test Method B' (described in the Examples Section).

Any suitable plasma treatment can be used. Plasma treatment refers to the use of an electrical discharge applied to a liquid or gaseous medium. Plasma treatment can be carried out using either atmospheric or sub-atmospheric conditions, for example, as described in US 2019/0134243 A1 (Nelson et al.). Exposure of the collagen matrix can take place either in direct contact with the plasma or in a downstream location. Energy is applied in the form of coupled electrical power to the liquid or gaseous medium creating new reactive species to interact with the collagen matrix. Typical plasma treatments create oxidizing and/or acidic species. In some embodiments, the applied energy density is greater than 0.05 eV/molecule of the gas passing through the plasma.

Low pH collagen dressing can provide advantages for wound healing, for example, it can affect the function of some enzymes, such as proteases, which if unchecked lead to the breakdown of tissue, perpetuating the wounded state.

The collagen matrix can be then dehydrated using any number of methods. This step may be referred to as dehydrating, drying or desiccating, all of which refer herein to the process of removing water content from the mixture or gel as possible. Dehydration can therefore be accomplished using heat, vacuum, lyophilization, desiccation, filtration, air-drying, critical point drying, and the like. In some embodiments, lyophilization may be preferred since the resulting collagen material is less likely to swell once in contact with an aqueous solution. However, the oven-dried gel sheets were observed to be more transparent and more uniform than the lyophilized sheets. The dehydration step may occur over a range of time, depending on the particular method used and the volume of mixture or gel. For example, the step may last for a few minutes, a few hours, or a few days. The present disclosure is not intended to be limited in this regard. In some embodiments, the dehydrated gel will swell when combined with water (i.e. rehydrated).

The dehydrated collagen matrix typically has a water content of at least 1, 2, 3, 4, or 5 wt-%. In some embodiments, the dehydrated collagen matrix has a water content of at least about 10, 15, or 20 wt-%.

The collagen matrix is dehydrated to reduce the water content and thereby increase the collagen concentration. Higher collagen concentrations generally promote healing more rapidly than lower collagen concentrations. The collagen matrix, prior to dehydration typically comprises 0.6 wt% - 6 wt% collagen. After dehydration, the collagen matrix typically comprises more than 50, 55, 60, 65, 70, 75, 80, 85, or 90 wt.-% collagen. After dehydration, the collagen matrix typically comprises no greater than 99 wt.-% and in some embodiments no greater than 95, 90, 85, 80, 70, or 60 wt.-% collagen.

The (e.g. dehydrated) collagen matrix can include various additives. Examples of additives can include any of antimicrobial agents, anti-inflammatory agents, topical anesthetics (e.g., lidocaine), other drugs, growth factors, polysaccharides, glycosaminoglycans. If an additive is included, it should be included at a level that does not interfere with the activity of the collagen matrix with respect to promoting healing of the wound. Additional additives can include those listed in WO 2016/160541 (Bjork et al.), expressly incorporated herein by reference in their entirety into this disclosure.

The (e.g. dehydrated) collagen matrix can have various physical forms, for example, hydrogel, a gel, a film, a paste, a sheet, a foam, or particles. The collagen hydrogel is typically sufficiently flowable at a temperature ranging from 0 °C to 37°C such that the collagen hydrogel takes the physical form of the container surrounding the collagen hydrogel. For, example if the collagen hydrogel is cast into a rectangular pan, the collagen hydrogel forms into a sheet. Thus, the collagen hydrogel can be cast into various shaped containers or in other words molded to provide (e.g. dehydrated) hydrogel of various shapes and sizes.

In one embodiment, the (e.g. dehydrated) collagen hydrogel may be provided as a foam. In another embodiment, the (e.g. dehydrated) collagen hydrogel may be provided as particles. For example, a sheet of (e.g. dehydrated) collagen hydrogel can be (e.g. laser or die) cut into pieces having various shapes and sizes. In another example, the dehydrated hydrogel may be ground, pulverized, milled, crushed, granulated, pounded, and the like, to produce powder.

In some embodiments, the collagen matrix can include one or more compounds that promote crosslinking of collagen. Suitable compounds for promoting crosslinking include for example formaldehyde, glutaraldehyde, genipin, 1,4-butanediol diglycidyl ether (BDDGE), 1-ethyl-3-(3-dimethylaminopropyl)carbodiimide (EDC), N-hydroxysuccinimide (NHS) and analogs thereof. In one embodiment, the crosslinking compound is EDC. In another embodiment, the crosslinking compounds are EDC and NHS.

In other embodiments, (e.g. dehydrated) collagen matrix can be admixed with natural or chemically modified and synthetic biological carrier materials. In typical embodiments, (e.g. dehydrated) collagen hydrogel particles are provided on or within a carrier layer at a coating weight that is sufficient to provide the desired effect (e.g. promoting wound re-epithelialization). In some embodiments, the coating weight of the (e.g. dehydrated) collagen hydrogel particles is typically at least 0.2, 0.5 or 1 milligram per cm² and typically no greater than 20, 10 or 5 milligrams per cm².

The (e.g. dehydrated) collagen hydrogel composition described herein may be utilized as a wound dressing article. The wound dressing article described herein comprises a (e.g. dehydrated) collagen matrix in a suitable physical form such as a sheet (i.e. film), foam sheet, or collagen matrix disposed on or within a carrier layer. Thus, the (e.g. dehydrated) collagen matrix can be provided in various forms as a continuous or discontinuous layer.

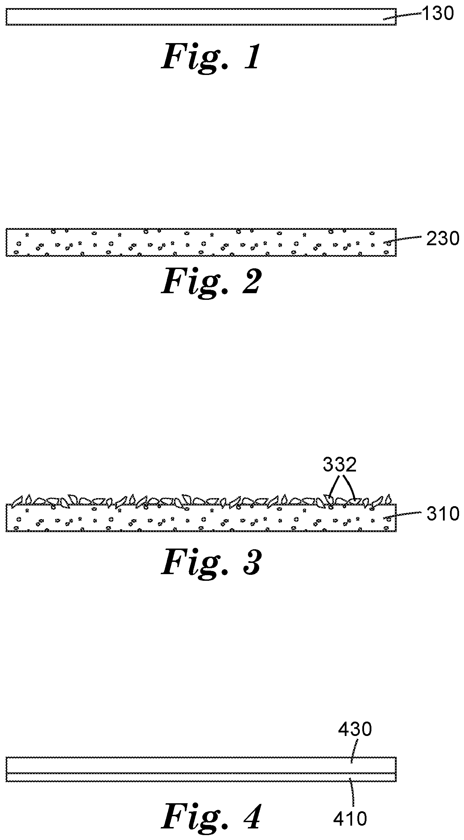

In some embodiments, the (e.g. dehydrated) collagen matrix is formed prior to combining the (e.g. dehydrated) collagen matrix with a carrier material or carrier layer. In other embodiments, a carrier layer can be combined with the aqueous solution comprising collagen,. For example, a fibrous (e.g. woven or nonwoven) substrate may be placed in a rectangular pan prior to adding the collagen thereby forming a sheet of collagen matrix having a fibrous scrim embedded within the hydrogel. FIGS. 1-4 as follow illustrative some typical wound dressings articles.

FIG. 1 illustrates an embodiment of a collagen matrix article, suitable for use as a wound dressing. The collagen matrix article includes a (e.g. flexible) sheet 130 comprising or consisting of the (e.g. dehydrated) collagen gel composition.

FIG. 2 illustrates another embodiment of a collagen matrix article, suitable for use as a wound dressing. The collagen article includes a sheet of foam 230 comprising or consisting of the (e.g. dehydrated) collagen gel composition. The foam may having various other shapes formed for example by molding the collagen hydrogel composition (e.g. prior to hydrating) or cutting the foam into pieces after it is formed.

The collagen matrix sheet articles, such as illustrated in FIGS. 1 and 2 typically have a thickness of at least 10 µm, 15 µm or 20 µm and typically no greater than 5 mm, 4 mm, 3mm, 2 mm, 1 mm, 0.5 mm, or 0.25 mm. In some embodiments, the thickness is no greater than 200, 150, 100, 75, or 60 microns. The basis weight of the collagen matrix sheet typically ranges from 2 to 10, 15, 20, 25 or 30 mg/cm².

The collagen matrix concentration of the sheet article is the same as the (e.g. dehydrated) collagen hydrogel as previously described.

FIG. 3 illustrates another embodiment of a collagen matrix article, suitable for use as a wound dressing. The collagen matrix article includes a carrier sheet of collagen sheet containing foam 230 or foam lacking collagen 310. The foam 230 or 310 further comprises a plurality of collagen particles 332 comprising the (e.g. dehydrated) collagen hydrogel disposed on and/or within the pores of the wound-facing surface of the foam. The particles may be collagen microbeads, collagen microcarriers, or collagen powder.

Each of the embodiments of FIGS 1-3 may further comprise a carrier layer disposed on a major surface of the collagen matrix sheet article. A carrier layer is typically disposed on the opposing major surface as the wound-facing surface. For example, FIG. 4 illustrates an embodiment of a collagen article, suitable for use as a wound dressing. The collagen article includes a sheet 430 comprising or consisting of the (e.g. dehydrated) collagen gel composition (e.g. 130, 230, or 310 together with 332) and a carrier layer 410.

In some embodiments, carrier layer 410 is a release liner. The release liner carrier may be disposed on the opposing major surface of both major surfaces (not shown) such that the collagen containing sheet is between the release liner layers.

Various release liners are known such as those made of (e.g. kraft) papers, polyolefin films such as polyethylene and polypropylene, or polyester. The films are preferably coated with release agents such as fluorochemicals or silicones. For example, U.S. Pat. No. 4,472,480 describes low surface energy perfluorochemical liners. Examples of commercially available silicone coated release papers are POLYSLIK^{™}, silicone release papers available from Rexam Release (Bedford Park, Ill.) and silicone release papers supplied by LOPAREX (Willowbrook, Ill.). Other non-limiting examples of such release liners commercially available include siliconized polyethylene terephthalate films commercially available from H. P. Smith Co. and fluoropolymer coated polyester films commercially available from 3M under the brand "ScotchPak^{™}" release liners.

In other embodiments, the carrier layer 410 may comprise a variety of other (e.g. flexible and/or conformable) carrier materials such as polymeric films and foams as well as various nonwoven and woven fibrous materials, such as gauze. In some embodiments, the carrier layer is absorbent, such as an absorbent foam. In other embodiments, the carrier layer is non-absorbent, such as a polymeric film.

In some embodiments, a method of treating a wound with an article of the disclosure is described but not claimed.

The method of treatment involves covering at least a portion of the wound with the article. At least a portion of the wound surface or wound fluid can be in contact with the collagen matrix portion of the article. In some embodiments, the method of treatment changes the pH of the wound environment. In some embodiments, the method of treatment decreases the pH of the wound environment. In some embodiments, the method of treatment decreases the pH of the wound fluid.

The wound to be treated by the method can be an open wound of the skin that exposes underlying body tissue. An open wound typically contains a wound fluid. Open wounds that can be treated by the method include acute wounds and chronic wounds.

The wound environment of a chronic wound (including the wound fluid) is often alkaline with a pH ranging from about 7.2-9. Wounds having an alkaline pH are known to have lower rates of healing than wounds having an acidic pH.

Open wounds that can be treated by the method include wounds to the skin from trauma (for example avulsions, incisions, and lacerations); wounds to the skin from pressure (for example pressure ulcers); and wounds to the skin from disease (for example venous ulcers, diabetic foot ulcers, and diabetic leg ulcers). In some embodiments, the wound environment is the exposed area of an open wound.

In some embodiments, the method of treatment changes the pH of the wound environment from alkaline to acidic. In some embodiments, the method of treatment changes the pH of the wound environment from greater than pH 7 to less than pH 7. In some embodiments, the method of treatment changes the pH of the wound environment from greater than pH 7 to either pH 3-6.8, pH 4-6.8, pH 4.5-6.8, pH 4-6.5, or pH 4.5-6.5.

In some embodiments, the method of treatment changes the pH of the wound fluid from alkaline to acidic. In some embodiments, the method of treatment changes the pH of the wound fluid from greater than pH 7 to less than pH 7. In some embodiments, the method of treatment changes the pH of the wound fluid from greater than pH 7 to either pH 3-6.8, pH 4-6.8, pH 4.5-6.8, pH 4-6.5, or pH 4.5-6.5.

In some embodiments, the method of treatment causes the pH of the wound environment to be from 3-6.9. In some embodiments, the method of treatment causes the pH of the wound environment to be from 3-6.8. In some embodiments, the method of treatment causes the pH of the wound environment to be from pH 4-6.8. In some embodiments, the method of treatment causes the pH of the wound environment to be from pH 4.5-6.8. In some embodiments, the method of treatment causes the pH of the wound environment to be from pH 4-6.5.

In some embodiments, the method of treatment causes the pH of the wound fluid to be from 3-6.9. In some embodiments, the method of treatment causes the pH of the wound fluid to be from 3-6.8. In some embodiments, the method of treatment causes the pH of the wound fluid to be from pH 4-6.8. In some embodiments, the method of treatment causes the pH of the wound fluid to be from pH 4.5-6.8. In some embodiments, the method of treatment causes the pH of the wound fluid to be from pH 4-6.5.

Also described but not claimed is a method of using a wound dressing article, with the method comprising contacting at least a portion of the collagen matrix of a wound dressing article of the present disclosure with an exposed surface of a wound. The wound dressing article can have a collagen matrix with a pH value from 2-6. The wound can be an open wound. The wound can be a chronic wound. In some embodiments the method of using a wound dressing article can cause the pH of the wound environment to change from alkaline to acidic. In some embodiments the method of using a wound dressing article can cause the pH of the wound environment to decrease. In some embodiments the method of using a wound article can cause the pH of the wound environment to be from 3-6.9, from 3-6.8, from 3.5-6.8, from 4-6.9, from 4-6.8, from 4.5-6.8, from 4-6.5, or from 4.5-6.5.

Further described but not claimed is a method of using a wound dressing article, with the method comprising contacting at least a portion of the collagen matrix of a wound dressing article of the present disclosure with the wound fluid of a wound. The wound can be an open wound. The wound can be a chronic wound. The wound dressing article can have a collagen matrix with a pH value from 2-6. In some embodiments the method of using a wound dressing article can cause the pH of the wound fluid to change from alkaline to acidic. In some embodiments the method of using a wound dressing article can cause the pH of the wound fluid to decrease. In some embodiments the method of using a wound article can cause the pH of the wound fluid to be from 3-6.9, from 3-6.8, from 3.5-6.8, from 4-6.9, from 4-6.8, from 4.5-6.8, from 4-6.5, or from 4.5-6.5.

The pH of a wound environment or wound fluid can be measured using pH paper, a pH electrode, a pH microelectrode, or any other suitable pH sensor.

The following working examples are intended to be illustrative of the present disclosure and not limiting.

### EXAMPLES

### Materials

Collagen type I from bovine calf skin (product #C857) was obtained from Elastin Products Company, Owensville, MO.
Human Dermal Fibroblasts, adult (HDFa) were obtained as GIBCO brand product #C0135C from Thermo Fisher Scientific, Waltham, MA.
Dulbecco's Modified Eagle Medium (DMEM) was obtained as GIBCO brand product #11965092 from Thermo Fisher Scientific, Waltham, MA.
Dulbecco's Modified Eagle Medium Complete (DMEM-C) was prepared by supplementing commercial DMEM with 10% serum and 1% penicillin/streptomycin.
1X Phosphate Buffered Saline (1X PBS) with a pH of 7.4 was obtained as GIBCO brand product #10010023 from Thermo Fisher Scientific, Waltham, MA.
1-Ethyl-3-(3-dimethylaminopropyl)carbodiimide (EDC); N-Hydroxysuccinimide (NHS); and resazurin sodium salt were obtained from the Sigma-Aldrich Corporation, St. Louis, MO.

### Methods

### Plasma Method A. Atmospheric Plasma Treatment of a Collagen Sample

Collagen samples were treated using an atmospheric plasma system of the design described in in United Sates Patent Application No. 20190134243. After the plasma generator, the effluent was transported through a six foot (1.83 meter) length of polytetrafluoroethylene (PTFE) tubing with a 1/8 inch (3.17 millimeter) diameter inner lumen. The lumen was adapted with a stainless steel adaptor to a 7/8 inch (22.22 mm) inner diameter (ID) PTFE sample tube that was eight inches (20.32 cm) in length. The sample tube was reduced to 3/16 inch (4.76 mm) on the atmospheric side. Collagen samples were loaded into the middle of the sample tube. After the samples were loaded in the sample tube, room air was introduced to the plasma electrode at a rate of 3 standard liter per minute (SLM). The gas precursor was humidified with vaporized water to 40% relative humidity (RH) at room temperature. Power was coupled to the plasma electrode from a 12 kHz ac power supply with a voltage of 3.6 kV and a total power of 85 W for a specified exposure time. At the end of the exposure, the power was turned off and samples were removed from the system.

### Plasma Method B. Sub-Atmospheric (Vacuum) Plasma Treatment of a Collagen Sample

Collagen samples were treated in an oxygen plasma using a Plasma-Therm 3032 batch plasma reactor (obtained from Plasma-Therm LLC, St. Petersburg, FL). The instrument was configured for reactive ion etching with a 26 inch (66 cm) lower powered electrode and central gas pumping. The chamber was pumped with a roots type blower (model EH1200 obtained from Edwards Engineering, Burgess Hill, UK)) backed by a dry mechanical pump (model iQDP80 obtained from Edwards Engineering). The RF power was delivered by a 3kW, 13.56 Mhz solid-state generator (RFPP model RF30S obtained from Advanced Energy Industries, Fort Collins, CO). The system had a nominal base pressure of 5 mTorr. The flow rates of the gases were controlled by MKS flow controllers (obtained from MKS Instruments, Andover, MA).

Samples were fixed on the powered electrode of the plasma reactor. After pumping down to the base pressure, oxygen was introduced at 500 standard cubic centimeters per minute (SCCM). Once the gas flow stabilized in the reactor, rf power (1000 watts) was applied to the electrode to generate the plasma. The plasma was ignited for the specified treatment time. Following completion of the plasma treatment, the chamber was vented to the atmosphere and the sample was removed from the chamber. Both sides of the collagen sample were treated. Treatment of the second side involved the same steps as described above with the sample flipped over on the powered electrode.

### pH Test Method A. pH Measurement of a Collagen Sample in 1X PBS

A 10 mg/mL suspension of the collagen sample was prepared in 1X phosphate buffered saline (1X PBS). The pH of the 1X PBS before the addition of the collagen sample was 7.4. The 1X PBS was maintained at 25 °C and the collagen sample was completely immersed in the 1X PBS media. Following immersion for one minute, the pH value of the liquid was measured using a calibrated pH meter.

### pH Test Method B. pH Measurement of a Collagen Sample in Water

A 10 mg/mL suspension of the collagen sample was prepared in distilled water. The pH of the distilled water before the addition of the collagen sample was 6.8-7.2. The water was maintained at 25 °C and the collagen sample was completely immersed in the water. Following immersion for 20 minutes, the pH value of the water was measured using a calibrated pH meter.

### Examples

### Example 1.

Collagen type I from bovine calf was dissolved in 20 mM acetic acid at a concentration of 7.5 mg/mL. A 12-14 mL aliquot of the solution was cast into a 6.3 cm inner diameter plastic tray (pre-treated with Rocket Release #E302 food grade release agent, obtained from Stoner Molding Solutions, Quarryville, PA ) and then frozen at negative 20 °C for at least one hour. The frozen sample was lyophilized to provide the collagen product as a conformable, porous matrix. The product had variable thicknesses of about 1-7 mm. Lyophilizations were conducted using a VirTis Advantage Plus EL-85 Freeze Dryer (SP Scientific, Warminster, PA).

A first plasma treatment according to Plasma Method A (described above) was applied to a sample of the collagen product. The plasma exposure time was 300 seconds. The resulting collagen sample was submitted to a second plasma treatment according to Plasma Method B (described above). Each side of the sample was plasma treated for 60 seconds.

### Example 2.

The same procedure as described in Example 1 was followed with the exception that the plasma exposure time for the first plasma treatment was 900 seconds, instead of 300 seconds.

### Example 3. pH Measurements of Collagen Samples Prepared by Examples 1-2

The pH of each sample prepared in Examples 1-2 was measured according to pH Test Method A (described above). The pH of the liquid was measured using a VWR SYMPHONY pH\conductivity meter (model# B30PCI, obtained from the VWR Corporation, Radnor, PA). The results are presented in Table 1. The results show that the plasma treated samples significantly lowered the pH of the 1X PBS media.

**Table 1. Measured pH after Immersion of Collagen**

| Sample in 1X PBS for 1 Minute (pH Test Method A) | |
|---|---|
| Collagen Sample | Measured pH of Liquid Media |
| Example 1 | 4-5 |
| Example 2 | 3 |

### Example 4.

Collagen type I from bovine calf skin was dissolved in 20 mM acetic acid at a concentration of 7.5 mg/mL. An aliquot of the solution (10 g) was cast into a 6.3 cm inner diameter plastic tray (pre-treated with Rocket Release #E302 food grade release agent, obtained from Stoner Molding Solutions) and then frozen at negative 80 °C for at least 20 minutes. The frozen sample was lyophilized to provide the collagen product as a conformable, porous matrix. The product had variable thicknesses of about 1-7 mm. Lyophilizations were conducted using a VirTis Advantage Plus EL-85 Freeze Dryer (SP Scientific).

A first plasma treatment according to Plasma Method A (described above) was applied to a sample of the collagen product. The plasma exposure time was 300 seconds. The resulting collagen sample was submitted to a second plasma treatment according to Plasma Method B (described above). Each side of the sample was plasma treated for 60 seconds.

### Example 5.

Collagen type I from bovine calf skin was dissolved in 20 mM acetic acid at a concentration of 7.5 mg/mL. In a separate vial, a solution of 8 mg of 1-ethyl-3-(3-dimethylaminopropyl)carbodiimide (EDC) and 8 mg of N-hydroxysuccinimide (NHS) in less than 1 mL of distilled water was prepared. This solution was then added to a vial containing 10 g of the collagen solution. The resulting mixture was mixed a 1500 rpm (revolutions per minute) for 30 seconds and then cast into a 6.3 cm inner diameter plastic tray (pre-treated with Rocket Release #E302 food grade release agent, obtained from Stoner Molding Solutions). The cast sample was maintained at room temperature for 30 minutes and then frozen at negative 20 °C for at least 20 minutes. The frozen sample was lyophilized to provide the collagen product as a conformable, porous matrix. The product had variable thicknesses of about 1-7 mm. Lyophilizations were conducted using a VirTis Advantage Plus EL-85 Freeze Dryer (SP Scientific).

A first plasma treatment according to Plasma Method A (described above) was applied to a sample of the collagen product. The plasma exposure time was 300 seconds. The resulting collagen sample was submitted to a second plasma treatment according to Plasma Method B (described above). Each side of the sample was plasma treated for 60 seconds.

### Comparative Example A.

Collagen type I from bovine calf skin was dissolved in 20 mM acetic acid at a concentration of 7.5 mg/mL. An aliquot of the solution (10 g) was cast into a 6.3 cm inner diameter plastic tray (pre-treated with Rocket Release #E302 food grade release agent, obtained from Stoner Molding Solutions) and then frozen at negative 20 °C for at least 20 minutes. The frozen sample was lyophilized to provide the collagen product as a conformable, porous matrix. The products had variable thicknesses of about 1-7 mm. Lyophilizations were conducted using a VirTis Advantage Plus EL-85 Freeze Dryer (SP Scientific).

### Example 6. pH Measurements of Collagen Samples Prepared by Examples 4-5 and Comparative

### Example A

The pH of each sample prepared in Examples 4-5 and Comparative Example A was measured according to pH Test Method B (described above). The pH of the water was measured using a VWR SYMPHONY pH\conductivity meter (model# B30PCI, obtained from the VWR Corporation). The results are presented in Table 2. The results show that the plasma treated samples significantly lowered the pH of the water media.

**Table 2. Measured pH after Immersion of Collagen**

| Sample in Water for 20 Minutes (pH Test Method B) | |
|---|---|
| Collagen Sample | Measured pH of Water Media |
| Example 4 | 5.3 |
| Example 5 | 3.5 |
| Comparative Example A | 6.7 |

### Example 7. Cell Viability Assay

Human dermal fibroblasts (HDFa) were cultured in DMEM-C media and seeded into a 24-well tissue treated plates at 50,000 cells per well. Cells were allowed to adhere overnight. Individual collagen samples were prepared by die punching a section (5 mm diameter) from the collagen products of Examples 1 and 2. A single die punched sample was added to a well. A control well was prepared in which no die punched sample was added to the well. A negative control well was also prepared in which the original DMEM-C media from a well with adhered cells was removed and replaced with DMEM media. In addition, the negative control well did not contain a die punched sample. The cells were cultured for 24 hours and then evaluated for cell viability using a resazurin cell viability assay. The percent cell viability values were determined relative to the control well with the control well assigned a percent cell viability of 100%. The results are reported in Table 3.

**Table 3.**

| Sample From | Cell Viability (percent) |
|---|---|
| No Sample (Control) | 100% |
| Example 1 | 97.3% |
| Example 2 | 96.5% |
| No Sample (Negative Control) | 96.4% |

### Example 8. Cell Viability Assay

A single collagen sample selected from either Example 1 or 2 was added to a vial containing DMEM cell culture media at a 10 mg/mL concentration. Each sample was completely immersed in the media for 48 hours at 4 °C with gentle agitation. The media from each sample was then sterile filtered using a 0.22 micron filter.

Human dermal fibroblasts (HDFa) were cultured in DMEM-C media and seeded into a 24-well tissue treated plates at 50,000 cells per well. Cells were allowed to adhere overnight. The DMEM-C media in a well was then removed and replaced with sterile filtered media prepared from either Example 1 or Example 2. A control well was prepared by removing the original DMEM-C media and replacing it with fresh DMEM-C media. A negative control well was also prepared in which the original DMEM-C media was removed and replaced with DMEM media. The cells were cultured for 24 hours and then evaluated for cell viability using a resazurin cell viability assay. The percent cell viability values were determined relative to the control well with the control well assigned a percent cell viability of 100%. The results are reported in Table 4.

**Table 4.**

| Sample From | Cell Viability (percent) |
|---|---|
| No Sample (Control) | 100% |
| Example 1 | 99.5% |
| Example 2 | 91.7% |
| No Sample (Negative Control) | 77.6% |

Illustrative embodiments of this invention are discussed and reference has been made to possible variations within the scope of this invention. For example, features depicted in connection with one illustrative embodiment may be used in connection with other embodiments of the invention. These and other variations and modifications in the invention will be apparent to those skilled in the art without departing from the scope of the invention, and it should be understood that this invention is not limited to the illustrative embodiments set forth herein. Accordingly, the invention is to be limited only by the claims provided below.

## Claims

1. A method of making an article, comprising
dissolving collagen in an acid solution;
forming a collagen matrix; and
reducing pH value of the collagen matrix by a first plasma treatment;
wherein the article is a wound dressing.

2. The method of claim 1, wherein the first plasma treatment is substantially at atmospheric pressure.

3. The method of any of claims 1-2, wherein the first plasma treatment comprises introducing an input gas comprising oxygen, nitrogen, and water vapor.

4. The method of any of claims 1-3, further comprising a second plasma treatment after the first plasma treatment.

5. The method of any of claims 1-4, wherein the second plasma treatment comprises introducing an oxygen plasma at sub atmospheric pressure.

6. The method of any of claims 1-5, further comprising dehydrating the collagen solution.

7. The method of claim 6, wherein the dehydrating comprises freeze-drying, oven drying, critical point drying, or combination thereof.

8. The method of any of claims 1-7, further comprising adding the matrix on a substrate.

9. The method of any of claims 1-8, further comprising disposing the collagen matrix on or within a carrier.

## Patentansprüche

1. Ein Verfahren zum Herstellen eines Artikels, aufweisend
Auflösen von Kollagen in einer Säurelösung;
Ausbilden einer Kollagenmatrix; und
Reduzieren des pH-Werts der Kollagenmatrix durch eine erste Plasmabehandlung;
wobei der Artikel ein Wundverband ist.

2. Das Verfahren nach Anspruch 1, wobei die erste Plasmabehandlung im Wesentlichen bei atmosphärischem Druck erfolgt.

3. Das Verfahren nach einem der Ansprüche 1 bis 2, wobei die erste Plasmabehandlung ein Einführen eines Eingangsgases aufweist, aufweisend Sauerstoff, Stickstoff und Wasserdampf.

4. Das Verfahren nach einem der Ansprüche 1 bis 3, ferner aufweisend eine zweite Plasmabehandlung nach der ersten Plasmabehandlung.

5. Das Verfahren nach einem der Ansprüche 1 bis 4, wobei die zweite Plasmabehandlung das Einführen eines Sauerstoffplasmas bei unter atmosphärischem Druck aufweist.

6. Das Verfahren nach einem der Ansprüche 1 bis 5, ferner aufweisend ein Dehydratisieren der Kollagenlösung.

7. Das Verfahren nach Anspruch 6, wobei das Dehydratisieren ein Gefriertrocknen, ein Ofentrocknen, ein Kritischer-Punkt-Trocknen oder eine Kombination davon aufweist.

8. Das Verfahren nach einem der Ansprüche 1 bis 7, ferner aufweisend ein Hinzufügen der Matrix auf einem Substrat.

9. Das Verfahren nach einem der Ansprüche 1 bis 8, ferner aufweisend ein Anordnen der Kollagenmatrix auf einem oder innerhalb eines Trägers.

## Revendications

1. Procédé de fabrication d'un article, comprenant
la dissolution de collagène dans une solution acide;
la formation d'une matrice de collagène; et
la réduction d'une valeur de pH de la matrice de collagène par un premier traitement au plasma;
dans lequel l'article est un pansement.

2. Procédé selon la revendication 1, dans lequel le premier traitement au plasma s'effectue essentiellement à la pression atmosphérique.

3. Procédé selon l'une quelconque des revendications 1 à 2, dans lequel le premier traitement au plasma comprend l'introduction d'un gaz d'entrée composé d'oxygène, azote et vapeur d'eau.

4. Procédé selon l'une quelconque des revendications 1 à 3, comprenant en outre un second traitement au plasma après le premier traitement au plasma.

5. Procédé selon l'une quelconque des revendications 1 à 4, dans lequel le second traitement au plasma comprend l'introduction d'un plasma d'oxygène à une pression sub-atmosphérique.

6. Procédé selon l'une quelconque des revendications 1 à 5, comprenant en outre la déshydratation de la solution de collagène.

7. Procédé selon la revendication 6, dans lequel la déshydratation comprend lyophilisation, séchage au four, séchage au point critique ou une combinaison de ceux-ci.

8. Procédé selon l'une quelconque des revendications 1 à 7, comprenant en outre l'ajout de la matrice sur un substrat.

9. Procédé selon l'une quelconque des revendications 1 à 8, comprenant en outre la disposition de la matrice de collagène sur ou à l'intérieur d'un support.
